# EUROPEAN PATENT SPECIFICATION

(11) **EP 3 841 916 B1**
(45) Date of publication and mention of the grant of the patent: **02.03.2022**
(21) Application number: 20187135.7
(22) Date of filing: 22.07.2020
(51) Int. Cl.: A45F 3/08

(54) **POWERED BACKPACK DEVICE FOR ASSISTING HUMANS TO WALK WITH LOAD**
ANGETRIEBENE RUCKSACKVORRICHTUNG ZUR UNTERSTÜTZUNG VON MENSCHEN BEIM GEHEN MIT LAST
DISPOSITIF DE SAC À DOS MOTORISÉ POUR AIDER LES HUMAINS À MARCHER AVEC UNE CHARGE

(30) Priority: 23.12.2019 CN 201911336233
(43) Date of publication of application: 30.06.2021
(73) Proprietor: Huazhong University of Science and Technology, Wuhan Hubei 430074 (CN)
(72) Inventor: XIONG, Caihua, Wuhan Hubei 430074 (CN); ZHANG, Qinhao, Wuhan Hubei 430074 (CN); HE, Lei, Wuhan Hubei 430074 (CN); LIU, Chuang, Wuhan Hubei 430074 (CN)
(74) Representative: Straus, Alexander

(56) References cited:
- CN-A- 110 051 106
- US-A1- 2008 185 411

## Description

### TECHNICAL FIELD

The invention belongs to the technical field of powered drive devices, and more specifically, relates to a powered backpack device for assisting a human to walk with a load.

### DESCRIPTION OF RELATED ART

Carrying loads is a common activity in daily life of human beings and is particularly common for soldiers in marching, hiking, and the like, and the soldiers may carry at most 50-60 kg of heavy loads for up to several hours or even more than ten hours during marching, and it is often necessary to continuously walk with a few kilograms to tens of kilograms of heavy loads on the back for several hours during hiking. Carrying a heavy load for a long time will cause harm to physiological structures and functions of a human body and result in great energy consumption of the human body. Carrying the loads leads to increased metabolic cost of the human body, increased muscle activity and muscle fatigue, and limits the motion scope of the human beings. In the meantime, the risk of musculoskeletal damage may also be increased. The human beings carry loads in various ways such as hand-holding a load, carrying a load under an arm, hanging a load on the waist, binding a load on a leg and carrying a load on shoulders, and in the various ways for carrying loads, a backpack is the most efficient way for carrying loads. However, high energy consumption may be still caused by carrying loads by adopting a traditional backpack, and therefore, it is still possible to improve an existing way that the human beings carry loads. For such circumstances, it is necessary to develop a wearable powered backpack device capable of assisting a human to carry a load; and by controlling the vertical acceleration of the load, the energy consumption of the human body is reduced, and the risk of musculoskeletal injuries resulting from load carrying is reduced.

CN 110 051 106 A discloses a backpack comprising a loading mechanism that reduces the load caused by the walking pressure of a human body.

Compared with the traditional backpack, the powered backpack device for assisting the human to walk with the load controls the vertical motion of the load while controlling an acting force of the load applied to the human body by controlling the vertical acceleration of the load, thereby reducing metabolic energy consumption and relieving muscle fatigue and shoulder pressure during load carrying. The metabolic energy consumption may be reduced when a wearer uses the powered backpack device, which requires researchers to research the principle that the human body carries a load, a load carrying motion control strategy, precision control on the acceleration of the load, the design of lightweight wearable robots and the safety of human-computer interaction. On these aspects, deep research is still needed. At present, a powered backpack device for assisting a human to walk with a load, which is completely autonomous and may also help the researchers perform researches on the aspect that the human body carries a load, has not been provided.

### SUMMARY OF THE INVENTION

In view of the above defects or needs for improvement of the prior art, the invention provides a powered backpack device for assisting a human to walk with a load; and due to the layout of key components and an overall structure of the powered backpack device, an acceleration and position of the load in a vertical direction are controlled in a backpack in real time to realize closed-loop control on the vertical acceleration and the position, so that the excursion of load and energy consumption of a human body during walking are reduced.

More specifically, the present invention relates to a powered backpack device for assisting a human to walk with a load as defined in claim 1. Preferred features of the invention are set out in the dependent claims.

To achieve the above purpose, according to the invention, provided is a powered backpack device for assisting a human to walk with a load. The device includes a base plate and a load, a ball screw, an elastic rope, a data acquisition module, a motion control module, a power supply and a controller arranged on the base plate, wherein

The ball screw is arranged in the center of the base plate; the load is arranged on the ball screw and is driven by the motion control module to move up and down on the ball screw; one end of the elastic rope is fixedly arranged on the base plate, and the other end of the elastic rope is connected to the load by pulleys; the gravity of the load in a vertical direction is balanced by virtue of the traction of the elastic rope on the load; and

The power supply is connected to the data acquisition module and the motion control module to respectively supply power to the data acquisition module and the motion control module; the data acquisition module and the motion control module are simultaneously connected to the controller; when the human starts walking with the backpack device on the back, the device enters a working mode under which the data acquisition module acquires a vertical acceleration of the human trunk and transmits the acquired acceleration to the controller, and the controller sets a desired vertical acceleration of the load according to the acceleration and makes the motion control module to drive the load to move at the desired acceleration on the ball screw; and when the human stops walking, the device enters an idle mode under which the data acquisition module acquires the position of the load and transmits the position of the load to the controller, and the controller makes the load stay on the ball screw by virtue of the motion control module.

Further preferably, the data acquisition module includes a human acceleration sensor, a load acceleration sensor and a displacement sensor, the human acceleration sensor is configured to measure the vertical acceleration of the human trunk in walking, the load acceleration sensor is configured to measure the vertical acceleration of the load, and the displacement sensor is configured to measure the position of the load on the ball screw.

Further preferably, the data acquisition module further includes a plantar pressure sensor configured to measure a plantar pressure for dividing gait cycle so as to facilitate data analysis.

Further preferably, the device further includes a handheld mode switch, when the human starts walking, the switch is manually turned off to make the device enter a working mode, and when the human stops walking, the switch is manually turned off to make the device enter an idlemode.

Further preferably, the motion control module includes a motor driver and a servo motor, the motor driver is electrically connected to the controller to accept a signal from the controller, the servo motor is electrically connected to the motor driver, the servo motor shaft is connected to the load by coupler, ball screw and nut consequently, and the servo motor is configured to drive the load to move according to the signal of the motor driver.

Further preferably, the power supply includes a lithium battery and a voltage converter, and the voltage converter is configured to supply power to the data acquisition module and the motion control module after converting a voltage.

Further preferably, the device is further provided with a Bluetooth configured to transmit data in the controller to a display device so as to display various data in the device.

Further preferably, the base plate is provided with a hollow pattern by which the mass of the base plate is reduced.

Generally speaking, compared with the prior art, the above-mentioned technical solution conceived by the invention may achieve the following beneficial effects:
1. the backpack device provided by the invention includes the data acquisition module, the controller and the motion control module, all of which form a closed control module, so that the backpack detects the vertical acceleration of the human trunk in real time according to the acceleration sensors when the human is in walking, adaptively adjusts the motion of the load and keeps the desired vertical acceleration, thereby relieving the burden of the human during walking, effectively reducing the metabolic energy consumption of the human in walking and relieving muscle fatigue and shoulder pressure during load carrying;
2. the data acquisition module provided by the invention includes the plurality of sensors by which the accelerations of the load and the human trunk, the position of the load and the gait cycle of the human are sufficiently measured, the data when the human moves and stops is monitored, and the accurate data support is provided for controlling the motion form and position of the load, so that the control result is more precise; and
3. the overall control system provided by the invention is autonomous, the vertical acceleration of the load is measured as a feedback signal, the detection for the motion state of the load does not depend on external measurement equipment, and the device may be used outside so as to be wide in application range.

### BRIEF DESCRIPTION OF THE DRAWINGS

Fig. 1 is a schematic structural diagram of a powered backpack device constructed according to a preferred embodiment of the invention;
Fig. 2 is a schematic connection diagram of a control module constructed according to a preferred embodiment of the invention;
Fig. 3 is a schematic flow diagram of a working process constructed according to a preferred embodiment of the invention; and
Fig. 4 is a control block diagram of the working process constructed according to a preferred embodiment of the invention.

In all the accompanying drawings, the same reference numerals are used to denote the same elements or structures, wherein

1-power supply, 2-controller, 3-data acquisition module, 4-motion control module, 5-Bluetooth, 6-fastening clasp, 7-base plate, 8-elastic rope, 9-load, 10-pulley, 11-coupler, 12-nut, 13-ballscrew, 14-support block, 31-human acceleration sensor, 32-load acceleration sensor, 33-displacement sensor, 34-pressure sensor, 41-motor driver, and 42-servo motor.

### DESCRIPTION OF THE EMBODIMENTS

In order to make the purposes, technical solutions and advantages of the invention more comprehensible, the invention will be further described in detail below with reference to the accompanying drawings and embodiments. It should be understood that the specific embodiments described herein are only intended to explain the invention, rather than to limit the invention. In addition, the technical features involved in the various embodiments of the invention described below can be combined with each other as long as they fall within the scope of the invention as defined in the appended claims.

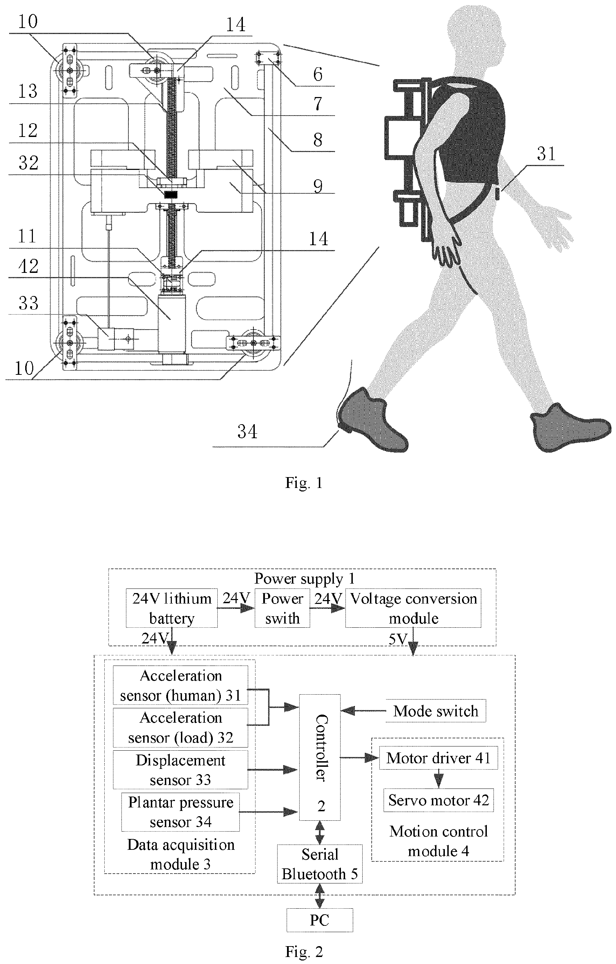

As shown in Fig. 1, a powered backpack device for assisting a human to walk with a load includes a fastening clasp 6, a base plate 7, an elastic rope 8, a load 9, pulleys 10, a coupler 11, a ball screw 13, a nut 12, support blocks 14, a power supply 1, a controller 2, a data acquisition module 3 and a motion control module 4; the power supply 1 includes a 24V lithium battery, a power switch and a voltage conversion module; the data acquisition module 3 includes a human acceleration sensor 31, a load acceleration sensor 32, a displacement sensor 33 and a plantar pressure sensor 34; and the motion control module 4 includes a motor driver 41 and a servo motor 42. In addition, the powered backpack device includes a mode switch and a human-computer interface part; and in the present embodiment, the base plate adopts a carbon fiber base plate with the strength being superior to that of a metal base plate.

The pulleys 10 and the fastening clasp 6 are fixedly arranged on the carbon fiber base plate 7; the elastic rope 8 is fixedly arranged between the fastening clasp 6 and the load 9 after bypassing the pulleys and is configured to balance the gravity of the load; the support blocks 14 are fixedly arranged on the carbon fiber base plate 7; the ball screw 13 is mounted between the two support blocks 14; the nut 12 is mounted on the ball screw 13 and is connected to the load 9; and the load 9 is composed of three copper blocks and is configured to simulate an actual load. Two detachable copper blocks in the copper blocks are adjusted, and thus, the mass of the load may be adjusted; the power supply1 is configured to convert power supplied by the 24V lithium battery into a voltage required by the data acquisition module 3, the motion control module 4 and acontroller 2, wherein the power switch controls the power supply of the overall system; and
as shown in Fig. 2, the data acquisition module 3 includes the human acceleration sensor 31, the load acceleration sensor 32, the displacement sensor 33 and the plantar pressure sensor 34, the motion control module includes a mode switch, the motor driver and the servo motor, and the load is jointly controlled by a driving force generated by the servo motor and a driven force generated by the elastic rope. The human acceleration sensor 31 is fixedly arranged on a waistband and is configured to measure a vertical acceleration of a human trunk, and the acceleration of the human trunk multiplied by a coefficient is used as a reference acceleration signal of the load; and the load acceleration sensor 32 is mounted on the load and is configured to measure a vertical acceleration of the load and use the acceleration as a feedback signal to be transmitted to the controller so that closed-loop control is realized. The displacement sensor 33 is mounted on the carbon fiber base plate, the end provided with a pulling rope is connected to the load, and the displacement sensor is configured to measure a displacement of the load relative to a backpack and transmit the displacement to the controller 2; and preferably, the plantar pressure sensor 34 is mounted at the bottom of an insole and is configured to measure a plantar pressure signal to divide a gait cycle and transmit the plantar pressure signal to the controller 2.

The motion control module 4 includes the motor driver 41 and the servo motor 42; the servo motor 42 is fixedly arranged on the support blocks 14, is connected with the coupler 11 and is configured to transmit the driving force to the load 9 by virtue of the coupler 11, the ball screw 13 and the nut 12 in sequence, thereby controlling the vertical acceleration of the load to have a certain relationship with the acceleration of the human trunk, and further reducing the metabolic energy consumption of the human body walking with the backpack; and the motor driver 41 works in a current mode and drives the servo motor 42 to generate a corresponding moment of force according to a received command current signal, and the moment of driving force is transmitted to the load by virtue of the ball screw.

The controller 2 is configured to read data of all the sensors, after calculation by using a control algorithm, the command current signal is transmitted to the motion control module 4, and the data is transmitted to an upper computer by virtue of a communication module; and after the motion control module receives the command current signal, the driving motor generates a corresponding moment of driving force, and then, the motion of the load is controlled by a transmission mechanism such as the ball screw.

The power switch may be configured to turn on or off a power supply of a control system and may cut off the power supply of the system in emergency circumstances, the voltage conversion module converts a 24V voltage of the lithium battery into 5V to be supplied to the control system, and the lithium battery directly supplies power to the motor and the driver.

The controller transmits the data to a computer by virtue of a serial Bluetooth, and the data is read and displayed by the upper computer programmed by using Matlab software in the computer.

The mode switch may select the system to be in a working mode or an idle mode; in the working mode, the acceleration of the load is controlled by adopting acceleration control; and in the idle mode, the position of the load is controlled to be stabilized on a certain position by adopting position constant-value control;
the controller 2 is connected to the motor driver 41 and is connected to the load acceleration sensor 32 and the human acceleration sensor 31 by a CAN bus, and the controller 2 operates the control algorithm to generate a command signal to be transmitted to the motion control module 3; and
a working principle of the backpack device provided by the invention will be specifically described below.

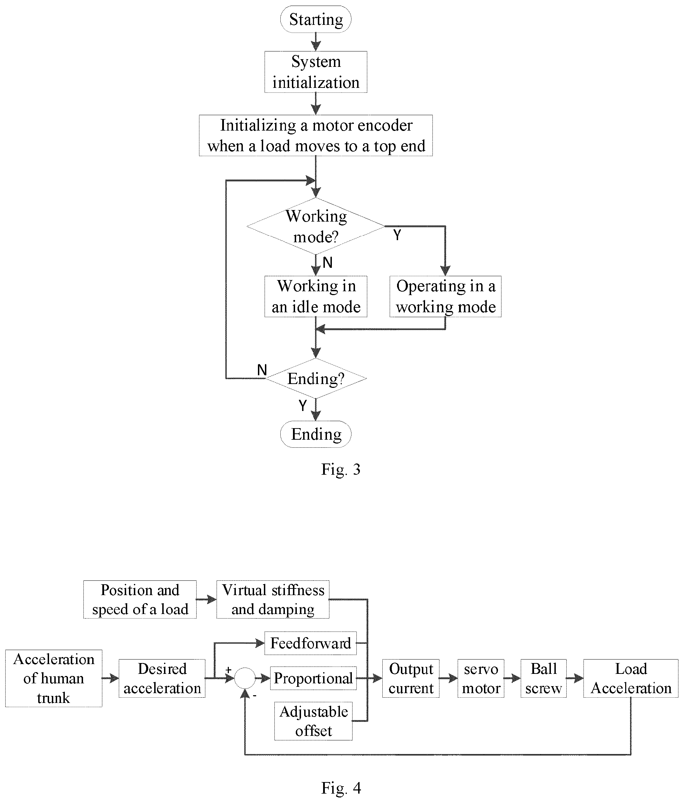

A specific process that the device is used to walk with a load is as follows:
As shown in Fig. 3 and Fig. 4, the acceleration sensors are fixedly arranged on a waistband. The powered backpack device is worn by virtue of a waistband and shoulder straps. The power switch and the mode switch are held in hands, the power switch is turned on to enter an initialized state, the servo motor 42 drives the load 9 to move to the top of the ball screw 13 by virtue of a transmission device, and an initial position value of the displacement sensor 33 is determined. Then, it is defaulted to enter the idle mode in which PID position control is used to detect the position of the load on the ball screw in real time according to the displacement sensor 33 and control the load to be stabilized on a certain fixed position. The mode switch is pressed down to enter the working state, the human acceleration sensor 31 acquires a vertical acceleration of a human trunk, and a desired acceleration of the load is generated according to the acceleration of the human trunk. The load acceleration sensor acquires the vertical acceleration of the load and takes the acceleration as a feedback signal, so that the closed-loop acceleration tracking control is realized. The displacement sensor 33 detects the position of the load relative to the backpack in real time, a speed is calculated in the controller 2, virtual stiffness and damping are generated according to the position and the speed and are compensated to the command current signal output by the controller, and thus, the phenomenon that the load moves to a boundary of the ball screw to result in collision may be avoided. The command current signal generated by the controller 2 operating the control algorithm is transmitted to the motor driver 41, the motor driver 41 works in a current control mode and controls the servo motor 42 to generate the corresponding moment of force, the generated moment of force is transmitted to the load by virtue of the coupler, the ball screw and the nut, and the vertical acceleration of the load is controlled under the combined action of the driving force generated by the servo motor 42 and the driven force generated by the elastic rope 8.

The mode switch is pressed down again to make the system work in the idle mode, the position of the load is detected by virtue of the displacement sensor 33, then, a detected result is transmitted to the controller 2, and the controller controls the load 9 to stay on a middle position of the backpack by virtue of the motion control module 4.

In the overall process, a user may stop the operation of the overall system by using the power switch, so that accidents are avoided, and the safety of the user is guaranteed.

When the computer is used to acquire the data, the data is read, displayed and stored by using the upper computer programmed by using the Matlab software. In the meanwhile, the computer may transmit control parameters to the controller 2 by virtue of the serial Bluetooth communication module 5 and change system parameters in real time. The data acquired by the plantar pressure sensor 34 is stored to divide the gait cycle of a human in walking so as to facilitate data analysis.

Those skilled in the art can easily understand that the above are only preferred embodiments of the invention and are not intended to limit the invention. Any modifications, equivalent replacements and improvements made should fall within the scope of the invention as defined in the appended claims.

## Claims

1. A powered backpack device for assisting a human to walk with a load comprising a base plate (7) and a load (9), a ball screw (13) an elastic rope (8), a data acquisition module (3), a motion control module (4), a power supply (1) and a controller (2)arranged on the base plate, wherein
the ball screw (13) is arranged in the center of the base plate (7); the load (9) is arranged on the ball screw and is driven by the motion control module (4) to move up and down on the ball screw (13); one end of the elastic rope (8) is fixedly arranged on the base plate, and the other end of the elastic rope (8) is connected to the load by pulleys (10); the gravity of the load in a vertical direction is balanced by virtue of the traction of the elastic rope on the load; and
the power supply (1) is connected to the data acquisition module (3) and the motion control module (4) to respectively supply power to the data acquisition module (3) and the motion control module (4); the data acquisition module (3) and the motion control module (4) are simultaneously connected to the controller (2); when the human starts walking with the backpack device on the back, the device enters a working mode under which the data acquisition module (3) acquires a vertical acceleration of the human trunk in motion and transmits the acquired acceleration to the controller (2), and the controller sets a desired vertical acceleration of the load according to the vertical acceleration of the human trunk and makes the motion control module to drive the load (9) to move at the desired acceleration on the ball screw(13); and when the human stops walking, the device enters an idle mode under which the data acquisition module acquires the position of the load and transmits the position of the load to the controller, and the controller makes the load stay on the ball screw by virtue of the motion control module.

2. The powered backpack device for assisting the human to walk with the load according to claim 1, **characterized in that** the data acquisition module (3) comprises a human acceleration sensor (31), a load acceleration sensor (32) and a displacement sensor (33), the human acceleration sensor is configured to measure the vertical acceleration of the human trunk in walking, the load acceleration sensor is configured to measure the vertical acceleration of the load, and the displacement sensor is configured to measure the position of the load on the ball screw.

3. The powered backpack device for assisting the human to walk with the load according to claim 1 or 2,**characterized in that** the data acquisition module (3) further comprises a plantar pressure sensor (34) which is configured to measure the plantar pressure for dividing a gait cycle of the human in walking so as to facilitate data analysis.

4. The powered backpack device for assisting the human to walk with the load according to any of claims 1 - 3, **characterized by** further comprising a handheld mode switch, when the human starts walking, the switch is manually turned off to make the device enter a working mode, and when the human stops walking, the switch is manually turned off to make the device enter an idle mode.

5. The powered backpack device for assisting the human to walk with the load according to any of claims 1 - 4,**characterized in that** the motion control module (4) comprises a motor driver (41) and a servo motor (42), the motor driver is electrically connected to the controller to accept a signal from the controller, the servo motor is electrically connected to the motor driver, the servo motor shaft is connected to the load by coupler, ball screw and nut consequently, and the servo motor is configured to drive the load to move according to the signal of the motor driver.

6. The powered backpack device for assisting the human to walk with the load according to any of claims 1 - 5,**characterized in that** the power supply (1) comprises a lithium battery and a voltage converter, and the voltage converter is configured to supply power to the data acquisition module or the motion control module after converting a voltage.

7. The powered backpack device for assisting the human to walk with the load according to any of claims 1 - 6,**characterized by** being further provided with a Bluetooth configured to transmit data in the controller to a display device so as to display various data in the device.

8. The powered backpack device for assisting the human to walk with the load according to any of claims 1 - 7,**characterized in that** the base plate is provided with a hollow pattern by which the mass of the base plate is reduced.

## Patentansprüche

1. Angetriebene Rucksackvorrichtung zur Unterstützung eines Menschen beim Gehen mit einer Last, umfassend eine Grundplatte (7) und eine Last (9), eine Kugelumlaufspindel (13), ein elastisches Seil (8), ein Datenerfassungsmodul (3), ein Bewegungssteuerungsmodul (4), eine Stromversorgung (1) und eine Steuerung (2), die auf der Grundplatte angeordnet sind, worin
die Kugelumlaufspindel (13) in der Mitte der Grundplatte (7) angeordnet ist; die Last (9) auf der Kugelumlaufspindel angeordnet ist und durch das Bewegungssteuermodul (4) angetrieben wird, um sich auf der Kugelumlaufspindel (13) auf und ab zu bewegen; worin ein Ende des elastischen Seils (8) fest auf der Grundplatte angeordnet ist und das andere Ende des elastischen Seils (8) mit der Last durch Umlenkrollen (10) verbunden ist; worin die Schwerkraft der Last in einer vertikalen Richtung durch die Zugkraft des elastischen Seils auf die Last ausgeglichen wird; und
die Stromversorgung (1) mit dem Datenerfassungsmodul (3) und dem Bewegungssteuerungsmodul (4) verbunden ist, um das Datenerfassungsmodul (3) bzw. das Bewegungssteuerungsmodul (4) mit Strom zu versorgen; worin das Datenerfassungsmodul (3) und das Bewegungssteuerungsmodul (4) gleichzeitig mit dem Steuergerät (2) verbunden sind; wobei wenn der Mensch mit der Rucksackvorrichtung auf dem Rücken zu gehen beginnt, die Vorrichtung in einen Arbeitsmodus eintritt, in dem das Datenerfassungsmodul (3) eine vertikale Beschleunigung des menschlichen Rumpfes in Bewegung erfasst und die erfasste Beschleunigung an die Steuerung (2) überträgt, und die Steuerung eine gewünschte vertikale Beschleunigung der Last entsprechend der vertikalen Beschleunigung des menschlichen Rumpfes einstellt und das Bewegungssteuerungsmodul veranlasst, die Last (9) anzutreiben, sich mit der gewünschten Beschleunigung auf der Kugelumlaufspindel (13) zu bewegen; und wenn der Mensch aufhört zu gehen, die Vorrichtung in einen Ruhezustand eintritt, in dem das Datenerfassungsmodul die Position der Last erfasst und die Position der Last an die Steuerung überträgt, und die Steuerung bewirkt, dass die Last aufgrund des Bewegungssteuerungsmoduls auf der Kugelumlaufspindel in Ruhe verbleibt.

2. Angetriebene Rucksackvorrichtung zur Unterstützung des Menschen beim Gehen mit der Last gemäß Anspruch 1, **dadurch gekennzeichnet, dass** das Datenerfassungsmodul (3) einen Sensor für menschliche Beschleunigung (31), einen Lastbeschleunigungssensor (32) und einen Versetzungssensor (33) umfasst, worin der Sensor für menschliche Beschleunigung ausgestaltet ist, die vertikale Beschleunigung des menschlichen Rumpfes beim Gehen zu erfassen, der Lastbeschleunigungssensor ausgestaltet ist, die vertikale Beschleunigung der Last zu erfassen, und der Verschiebungssensor ausgestaltet ist, die Position der Last auf der Kugelumlaufspindel zu erfassen.

3. Angetriebene Rucksackvorrichtung zur Unterstützung des Menschen beim Gehen mit der Last nach Anspruch 1 oder 2, **dadurch gekennzeichnet, dass** das Datenerfassungsmodul (3) ferner einen Fußsohlendrucksensor (34) umfasst, der ausgestaltet ist, den Fußsohlendruck zur Unterteilung eines Gangzyklus des Menschen beim Gehen zu erfassen, um die Datenanalyse zu erleichtern.

4. Angetriebene Rucksackvorrichtung zum Unterstützen des Menschen beim Gehen mit der Last nach einem der Ansprüche 1 bis 3, **dadurch gekennzeichnet, dass** sie ferner einen Handmodusschalter umfasst, der, wenn der Mensch zu gehen beginnt, manuell ausgeschaltet wird, um die Vorrichtung in einen Arbeitsmodus zu versetzen, und der, wenn der Mensch aufhört zu gehen, manuell ausgeschaltet wird, um die Vorrichtung in einen Ruhemodus zu versetzen.

5. Angetriebene Rucksackvorrichtung zum Unterstützen des Menschen beim Gehen mit der Last nach einem der Ansprüche 1 bis 4, **dadurch gekennzeichnet, dass** das Bewegungssteuermodul (4) einen Motortreiber (41) und einen Servomotor (42) umfasst, der Motortreiber elektrisch mit der Steuerung verbunden ist, um ein Signal von der Steuerung anzunehmen, der Servomotor elektrisch mit dem Motortreiber verbunden ist, die Servomotorwelle mit der Last durch eine Kugelumlaufspindel und eine Mutter verbunden ist, und der Servomotor ausgestaltet ist, die Last anzutreiben, sich entsprechend dem Signal des Motortreibers zu bewegen.

6. Angetriebene Rucksackvorrichtung zur Unterstützung des Menschen beim Gehen mit der Last nach einem der Ansprüche 1 bis 5, **dadurch gekennzeichnet, dass** die Stromversorgung (1) eine Lithiumbatterie und einen Spannungswandler umfasst, und der Spannungswandler ausgestaltet ist, das Datenerfassungsmodul oder das Bewegungssteuerungsmodul nach Umwandlung einer Spannung mit Strom zu versorgen.

7. Angetriebene Rucksackvorrichtung zur Unterstützung des Menschen beim Gehen mit der Last nach einem der Ansprüche 1 bis 6, **dadurch gekennzeichnet, dass** sie ferner mit Bluetooth ausgestattet ist, das konfiguriert ist, Daten in der Steuerung an eine Anzeigevorrichtung zu übertragen, um verschiedene Daten in der Vorrichtung anzuzeigen.

8. Angetriebene Rucksackvorrichtung zur Unterstützung des Menschen beim Gehen mit der Last nach einem der Ansprüche 1 bis 7, **dadurch gekennzeichnet, dass** die Grundplatte mit einem hohlen Muster versehen ist, durch das die Masse der Grundplatte reduziert wird.

## Revendications

1. Dispositif de sac à dos motorisé pour aider un humain à marcher avec une charge, comprenant une plaque de base (7) et une charge (9), une vis à billes (13), une corde élastique (8), un module d'acquisition de données (3), un module de commande de mouvement (4), une alimentation électrique (1) et un contrôleur (2) disposés sur la plaque de base, dans lequel
la vis à billes (13) est disposée au centre de la plaque de base (7); la charge (9) est disposée sur la vis à billes et est entraînée par le module de commande de mouvement (4) pour se déplacer vers le haut et vers le bas sur la vis à billes (13); une extrémité de la corde élastique (8) est disposée de manière fixe sur la plaque de base, et l'autre extrémité de la corde élastique (8) est reliée à la charge par des poulies (10); la gravité de la charge dans une direction verticale est équilibrée en vertu de la traction de la corde élastique sur la charge; et
l'alimentation électrique (1) est connectée au module d'acquisition de données (3) et au module de commande de mouvement (4) pour alimenter respectivement le module d'acquisition de données (3) et le module de commande de mouvement (4); le module d'acquisition de données (3) et le module de commande de mouvement (4) sont simultanément connectés au contrôleur (2); lorsque l'humain commence à marcher avec le dispositif de sac à dos sur le dos, le dispositif entre dans un mode de travail dans lequel le module d'acquisition de données (3) acquiert une accélération verticale du tronc humain en mouvement et transmet l'accélération acquise au contrôleur (2), et le contrôleur définit une accélération verticale souhaitée de la charge en fonction de l'accélération verticale du tronc humain et fait en sorte que le module de commande de mouvement entraîne la charge (9) pour qu'elle se déplace à l'accélération souhaitée sur la vis à billes (13); et lorsque l'humain s'arrête de marcher, le dispositif entre dans un mode de repos dans lequel le module d'acquisition de données acquiert la position de la charge et transmet la position de la charge au contrôleur, et le contrôleur fait en sorte que la charge reste sur la vis à billes grâce au module de commande de mouvement.

2. Dispositif de sac à dos motorisé pour aider un humain à marcher avec la charge selon la revendication 1, **caractérisé en ce que** le module d'acquisition de données (3) comprend un capteur d'accélération humaine (31), un capteur d'accélération de charge (32) et un capteur de déplacement (33), le capteur d'accélération humaine est configuré pour mesurer l'accélération verticale du tronc humain lors de la marche, le capteur d'accélération de charge est configuré pour mesurer l'accélération verticale de la charge, et le capteur de déplacement est configuré pour mesurer la position de la charge sur la vis à billes.

3. Dispositif de sac à dos motorisé pour aider un humain à marcher avec la charge selon la revendication 1 ou 2, **caractérisé en ce que** le module d'acquisition de données (3) comprend en outre un capteur de pression plantaire (34) qui est configuré pour mesurer la pression plantaire pour diviser un cycle de marche de l'humain en train de marcher de manière à faciliter l'analyse des données.

4. Dispositif de sac à dos motorisé pour aider un humain à marcher avec la charge selon l'une quelconque des revendications 1-3, **caractérisé en ce qu'**il comprend en outre un commutateur de mode portatif, lorsque l'humain commence à marcher, le commutateur est manuellement éteint pour faire entrer le dispositif dans un mode de travail, et lorsque l'humain arrête de marcher, le commutateur est manuellement éteint pour faire entrer le dispositif dans un mode de repos.

5. Dispositif de sac à dos motorisé pour aider un humain à marcher avec la charge selon l'une quelconque des revendications 1-4, **caractérisé en ce que** le module de commande de mouvement (4) comprend un pilote de moteur (41) et un servomoteur (42), le pilote de moteur est électriquement connecté au contrôleur pour accepter un signal du contrôleur, le servomoteur est électriquement connecté au pilote de moteur, l'arbre du servomoteur est connecté à la charge par un coupleur (vis à billes et écrou) en conséquence, et le servomoteur est configuré pour entraîner la charge à se déplacer selon le signal du pilote de moteur.

6. Dispositif de sac à dos motorisé pour aider un humain à marcher avec la charge selon l'une quelconque des revendications 1-5, **caractérisé en ce que** l'alimentation électrique (1) comprend une batterie au lithium et un convertisseur de tension, et le convertisseur de tension est configuré pour alimenter le module d'acquisition de données ou le module de commande de mouvement après avoir converti une tension.

7. Dispositif de sac à dos motorisé pour aider un humain à marcher avec la charge selon l'une quelconque des revendications 1-6, **caractérisé en ce qu'**il est en outre pourvu d'un Bluetooth configuré pour transmettre des données dans le contrôleur à un dispositif d'affichage de manière à afficher diverses données dans le dispositif.

8. Dispositif de sac à dos motorisé pour aider un humain à marcher avec la charge selon l'une quelconque des revendications 1-7, **caractérisé en ce que** la plaque de base est pourvue d'un motif creux par lequel la masse de la plaque de base est réduite.
